(19) Europäisches Patentamt
European Patent Office
Office européen des brevets

(11) **EP 2 905 619 A1**

(12) **EUROPEAN PATENT APPLICATION**

(43) Date of publication:
**12.08.2015 Bulletin 2015/33**

(51) Int Cl.:
***G01N 33/574*** *(2006.01)*

(21) Application number: **14154030.2**

(22) Date of filing: **05.02.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Dublin City University**
**Dublin 9 (IE)**

(72) Inventors:
• **Clynes, Martin**
**Dublin, D3 (IE)**

• **Dowling, Paul**
**Dublin, D3 (IE)**
• **Clarke, Colin**
**Dublin, D9 (IE)**

(74) Representative: **Purdy, Hugh Barry et al**
**PurdyLucey**
**Intellectual Property**
**6-7 Harcourt Terrace**
**D2 Dublin (IE)**

(54) **A method for monitoring response to therapy and recurrence of breast cancer**

(57)  A method of monitoring a breast cancer patient's response to treatment, or of detecting recurrence of breast cancer, the method comprising a step of assaying a plurality of samples obtained from the patient over a period of time for expression levels of a plurality of biomarkers including Cancer Antigen 15-3 (CA 15-3) and one of either Glutamate or 12-Hydroxyeicosatetraenoic acid (12-HETE), and detecting a change in expression levels of CA 15-3 and one (or both) of either Glutamate or 12-HETE over the time period, wherein detection of a decreased expression levels of CA 15-3 and one of either Glutamate or 12-HETE over the time period is indicative of a response to therapy and wherein detection of increased or stable expression levels of CA 15-3 and one of either Glutamate or 12-HETE over the time period is indicative of a non-response to breast cancer therapy.

CA15-3: Normal v Breast Cancer

Area under the curve = 0.809

Sensitivity

1-Specificity

**Figure 1A**

EP 2 905 619 A1

**(Cont. next page)**

Figure 1B

Figure 1C

## Description

### Field of the Invention

The present invention relates to a method of monitoring the response of an individual breast cancer to various therapies, and of diagnosing the recurrence of breast cancer in a patient by measuring the levels of a combination of at least 2 biomarkers.

### Background to the Invention

Treatment options for breast cancer are increasing all the time, including surgery, systemic therapy (chemotherapy, biological therapy, targeted therapy, hormonal therapy) and local therapy (surgery, radiation therapy) or a combination of these treatments. Therefore it is important to know, as early and accurately as possible, whether or not a particular treatment is working, so that if necessary an early switch can be made to a different regimen.

Tumour markers (TMs) are widely used in the management of patients with breast cancer, during therapy for metastatic disease and in conjunction with diagnostic imaging, history and physical examination. Cancer antigen 15-3 (CA 15-3) is a biomarker that is highly associated with breast cancer and is derived from the MUC1 gene product. CA 15-3 has been reported to be elevated in serum from 70-75% of metastatic breast cancers. A decrease in marker levels during treatment can indicate tumour response, whereas stable or increasing levels can indicate that the tumour is not responding to treatment or that the tumour is recurring.

However, CA 15-3 levels can also be raised due to the presence of other conditions. CA 15-3 levels can also be raised in normal, healthy individuals with no history of breast cancer. This marker is not recommended by physician organizations for use in the screening or detection of breast cancer. However given the absence of any better blood test, CA 15-3 is measured to aid physicians in the monitoring of treatment of women with breast malignancies and also to diagnose recurrence of the disease following first-line therapy.

The object of the subject application is to overcome at least one of the above-referenced problems.

### Summary of the Invention

It is known from the prior art that CA 15-3 is measured from serum samples of patients to aid physicians in the monitoring of treatment of invasive breast malignances and also to diagnose recurrence of the disease following first-line therapy. A decrease in CA 15-3 levels during treatment can indicate tumour response, whereas stable or increasing levels of CA 15-3 despite adequate treatment can indicate that the tumour is not responding to treatment or that the tumour is recurring. CA 15-3 is elevated in the serum/plasma of approximately 75% of women with metastasized breast cancer.

The problem with using CA 15-3 as a prognostic or diagnostic marker appears to be that CA 15-3 levels can also be elevated due to the presence of other conditions *i.e.* limited specificity and elevated levels can also be seen in healthy individuals. Further, only about 70-75% of patients with of breast cancer will show an elevated serum CA 15-3 level. The solution to the problem is provided by the invention presented herein, where by combining this widely used cancer marker CA 15-3 with Glutamate or 12-Hydroxyeicosatetraenoic acid (12-HETE), or a combination of all three, significantly increases the accuracy (sensitivity and specificity) of the test. A combination of both CA 15-3 and Glutamate were found to have an AUC value of 0.95 while a combination of CA 15-3 and 12-HETE had an AUC value of 0.94 for distinguishing control from metastatic breast cancer samples. The final combination of all three biomarkers, CA 15-3, Glutamate and 12-HETE had an AUC value of 0.96 thus representing the best combination of molecules for distinguishing normal/non-cancer samples from breast cancer samples. This represents a significant improvement in the ability to monitor the effectiveness of treatment compared with monitoring CA 15-3 alone which has an AUC value of 0.8 for distinguishing the normal/non-cancer group from the breast cancer group.

According to the present invention, there is provided, as set out in the appended claims, a method of monitoring a breast cancer patient's response to treatment, and of detection of recurrence of a breast cancer.

In one embodiment of the present application, there is provided a method of monitoring a breast cancer patient's response to treatment, the method comprising a step of:

assaying a plurality of samples obtained from the patient over a period of time for expression levels of a plurality of biomarkers including Cancer Antigen 15-3 (CA 15-3) and one of either Glutamate or 12-Hydroxyeicosatetraenoic acid (12-HETE), and
detecting a change in expression levels of CA 15-3 and one of either Glutamate or 12-HETE over the time period,

wherein detection of a decreased expression levels of CA 15-3 and one of either Glutamate or 12-HETE over the time period is indicative of a response to therapy and wherein detection of increased or stable expression levels of CA 15-3 and one of either Glutamate or 12-HETE over the time period is indicative of a non-response to breast cancer therapy.

In one embodiment of the present application, there is provided a method for determining recurrence of cancer in a breast cancer patient, the method comprising a step of:

assaying a plurality of samples obtained from the patient over a period of time for expression levels of a plurality of biomarkers including Cancer Antigen 15-3 (CA 15-3) and one of either Glutamate or 12-Hydroxyeicosatetraenoic acid (12-HETE), and

detecting a change in expression levels of CA 15-3 and one of either Glutamate or 12-HETE when compared to reference expression levels for the biomarkers,

wherein detection of increased expression levels of CA 15-3 and one of either Glutamate or 12-HETE when compared to the reference expression levels is indicative of a recurrence of cancer in the patient.

In one embodiment, the biomarkers include CA 15-3 and both Glutamate and 12-HETE, wherein detection of increased expression levels of CA 15-3, Glutamate and 12-HETE, when compared with reference expression levels, is indicative of a risk of recurrence of breast cancer.

In one embodiment, the biomarkers include CA 15-3 and both Glutamate and 12-HETE, wherein detection of stable or increased expression levels of CA 15-3, Glutamate and 12-HETE is indicative of a non-response to breast cancer therapy.

In one embodiment, the biomarkers include CA 15-3 and both Glutamate and 12-HETE, wherein detection of decreased expression levels of CA 15-3, Glutamate and 12-HETE is indicative of a response to breast cancer therapy.

In one embodiment, the methods described above have a discriminatory power (AUC value) of 0.94 or greater. Ideally, the methods have a discriminatory power (AUC value) of 0.95 or greater.

Preferably, the methods described above have a plurality of biomarkers which include CA 15-3 and both Glutamate and 12-HETE, and in which the methods have a discriminatory power (AUC value) of 0.96 or greater.

In one embodiment, the sample is selected from blood, serum, saliva, urine, cerebrospinal fluid, tissue, cells, breast cancer tumour specimen.

In one embodiment, the expression levels are assayed by immunohistochemistry (IHC), enzyme-linked immunosorbent assay (ELISA), and other antibody-based methods which could be, for example, colorimetric, flurimetric, potentiometric, or radiometric, or in the case of glutamate, an enzyme-based colorimetric assay.

In one embodiment, the method is carried out periodically after surgery and/or during first line therapy.

In one embodiment, the detection of a decreased expression level of CA 15-3 and one of either Glutamate or 12-HETE relative to reference expression levels for the biomarkers over the time period is indicative of a response to therapy and wherein detection of increased or stable expression levels of CA 15-3 and one of either Glutamate or 12-HETE relative to reference expression levels for the biomarkers over the time period is indicative of a non-response to breast cancer therapy.

In one embodiment of the present application, there is provided a method of treating or preventing further progression of breast cancer in a patient with a primary breast cancer, the method comprising the step of administering a second line therapy to the patient, wherein the patient is identified as having a non-responsive or a recurring breast cancer, using a method as described above.

In one embodiment of the present application, there is provided a system for monitoring response to therapy and recurrence of breast cancer in an individual, the system comprising:

a determination module configured to receive at least one test sample and perform at least one test analysis on the test sample to assay for expression of a plurality of biomarkers including CA 15-3 and one of either Glutamate or 12-HETE, or all three;

optionally, a storage system configured to receive and store the expression data generated by the determination system for CA 15-3 and one of either Glutamate or 12-HETE, or all three;

a comparison module for comparing the expression levels of the plurality of biomarkers with reference levels of the biomarkers and determining down-regulated, stable or increased expression of the biomarkers; and

a display module for displaying a content based in part on the data output from said comparison module, wherein when stable or increased expression of CA 15-3 and one of either Glutamate or 12-HETE, or all three, is determined, the content comprises a signal indicative of a lack of response to therapy and/or recurrence of breast cancer.

In one embodiment, the determination system comprises any quantitative method for quantitating specific antibody binding or a colorimetric detection apparatus.

In one embodiment, the discriminatory power, provided as an AUC value, for the monitoring of response to therapy between an individual with breast cancer and one without according to the invention is 0.90 or greater, preferably 0.91, 09.2, 0.93, or 0.94. More preferably, the AUC value is 0.95 or greater. Ideally, the AUC value is 0.96, 0.97, 0.98, 0.99 or 1.00.

Typically, the methods of the invention are suitable for patients on endocrine therapy.

Levels of accuracy for diagnosing recurrence of breast cancer, or of monitoring response to therapy using CA 15-3 in combination with glutamate and/or 12-HETE (or all three markers) significantly increase AUC values, for example from 0.8 to 0.96 when all three markers are used, which represents a significant improvement in sensitivity and specificity of the test, as well as illustrating a synergistic effect when combining all three biomarkers. The availability of biomarkers to monitor the effectiveness of treatment and switch to alternatives when resistance/toxicity is observed is very beneficial. This combination of biomarkers has utility in both of these areas.

A further advantage of the invention is that very frequent mammograms following diagnosis of and/or treatment for breast cancer can be avoided by monitoring the expression levels of CA 15-3 and one or both of Glutamate and 12 HETE.

## Brief Description of the Drawings

The invention will be more clearly understood from the following description of an embodiment thereof, given by way of example only, with reference to the accompanying drawings, in which:-

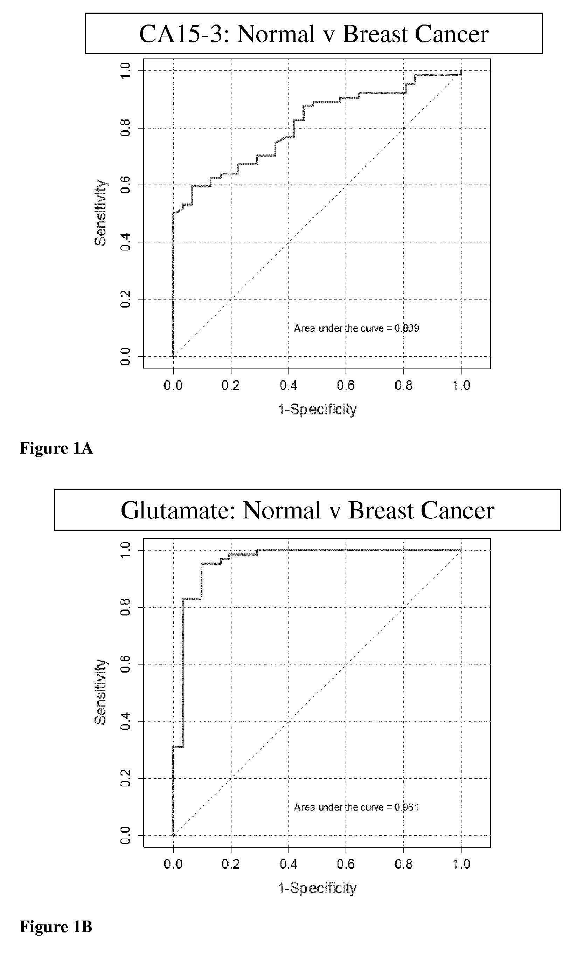

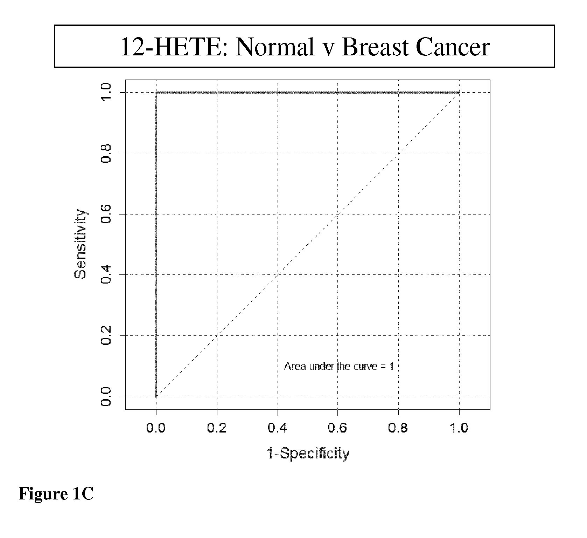

**Figure 1A-1C** illustrate AUC curves for normal v. breast cancer for all 3 markers CA 15-3, Glutamate and 12 HETE, individually.

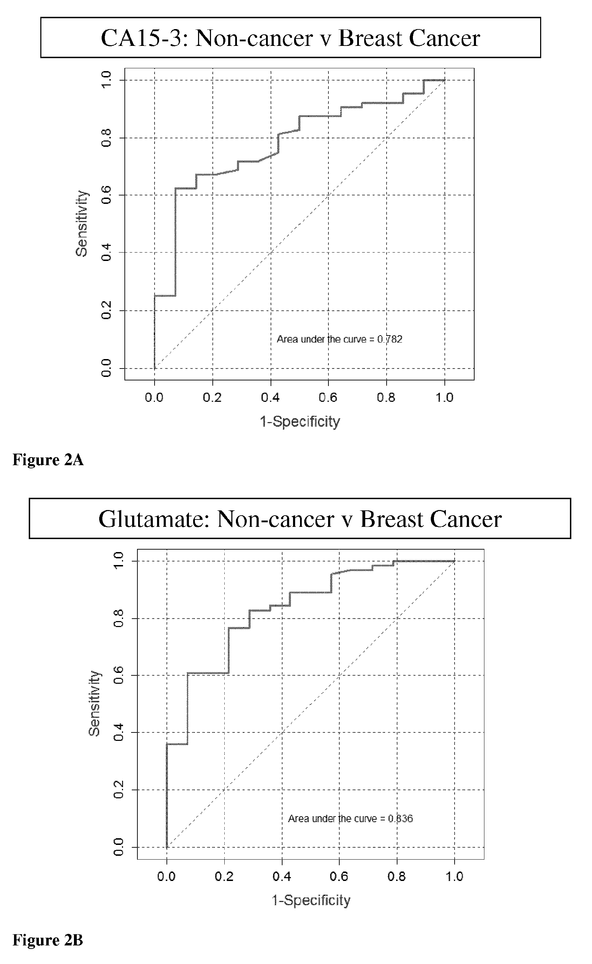

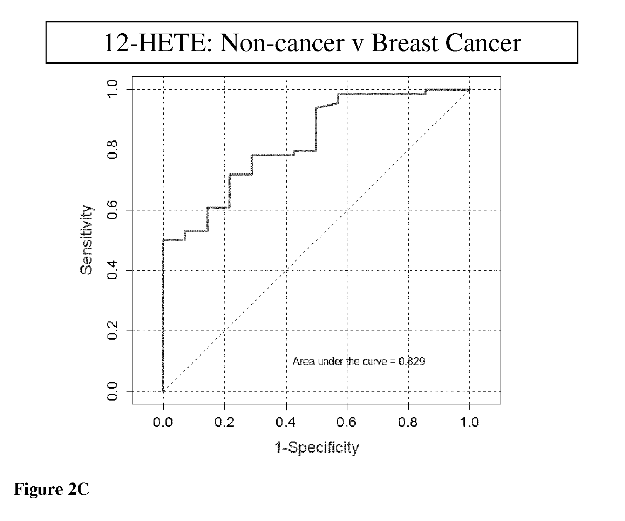

**Figure 2A-2C** illustrate AUC curves for non-cancer v. breast cancer for all 3 markers, CA 15-3, Glutamate and 12 HETE, individually.

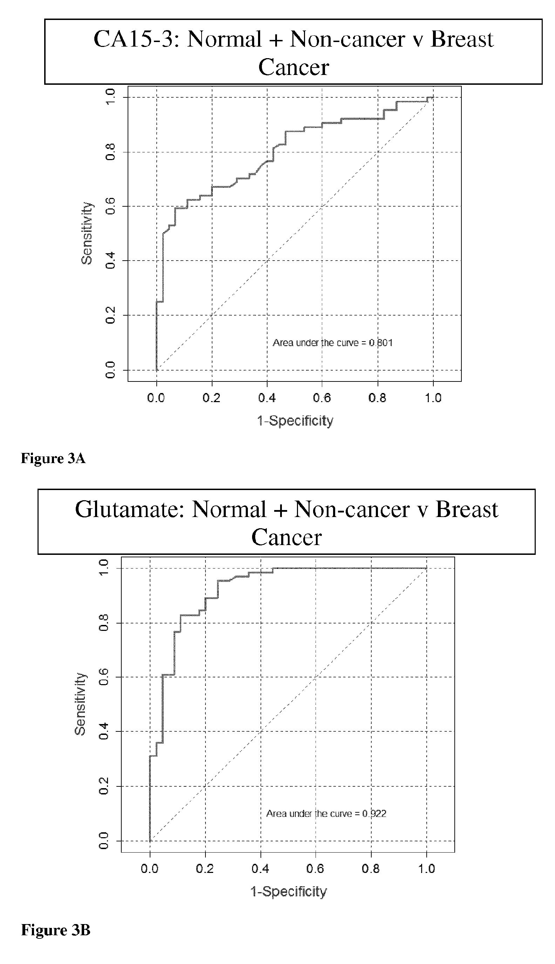

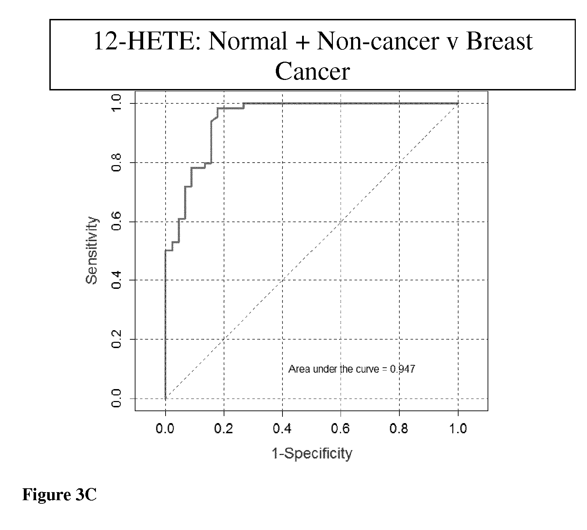

**Figure 3A-3C** illustrate AUC curves for normal and non-cancer v. breast cancer for all 3markers, CA 15-3, Glutamate and 12 HETE, individually.

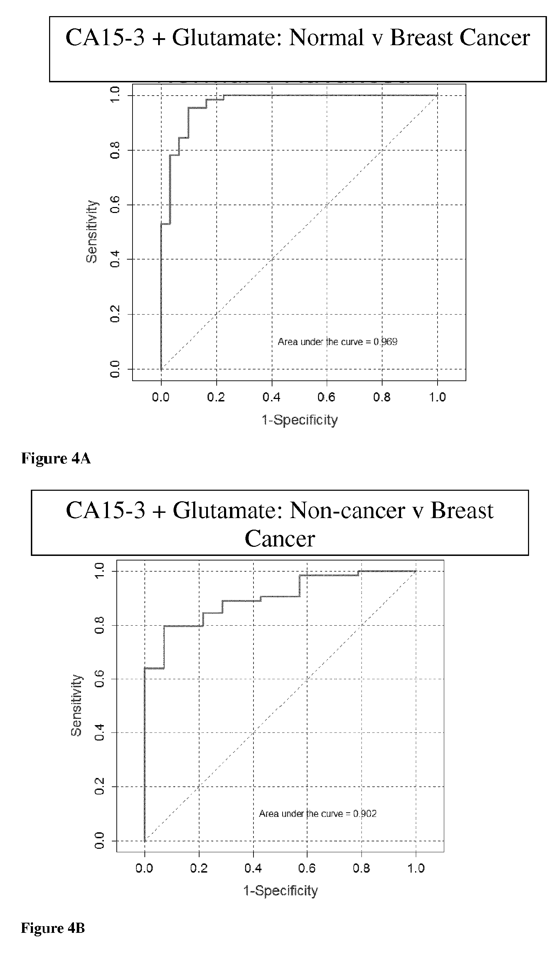

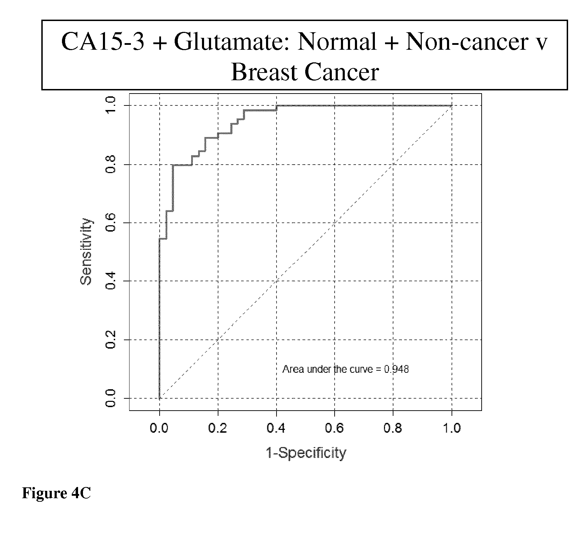

**Figure 4A-4C** illustrate AUC curves for (A) normal, (B) non-cancer, and (C) normal and non-cancer v. breast cancer for CA 15-3 and Glutamate combined.

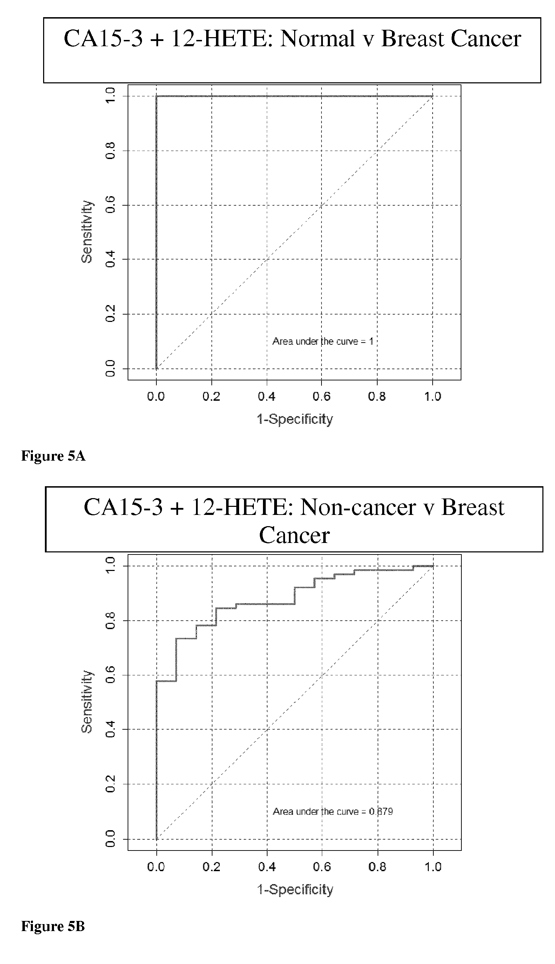

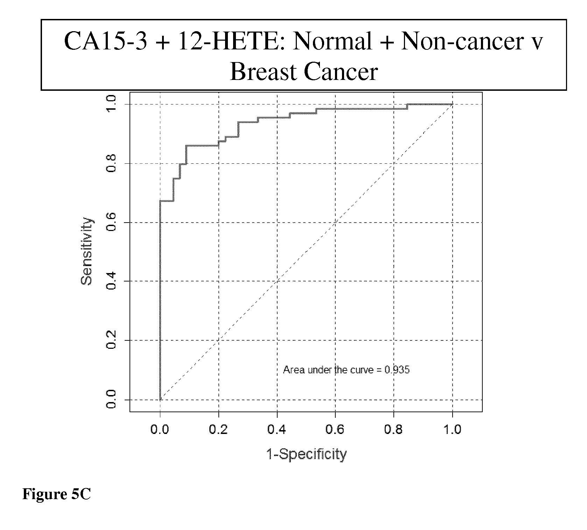

**Figure 5A-5C** illustrate AUC curves for (A) normal, (B) non-cancer, and (C) normal and non-cancer v. breast cancer for CA 15-3 and 12 HETE combined.

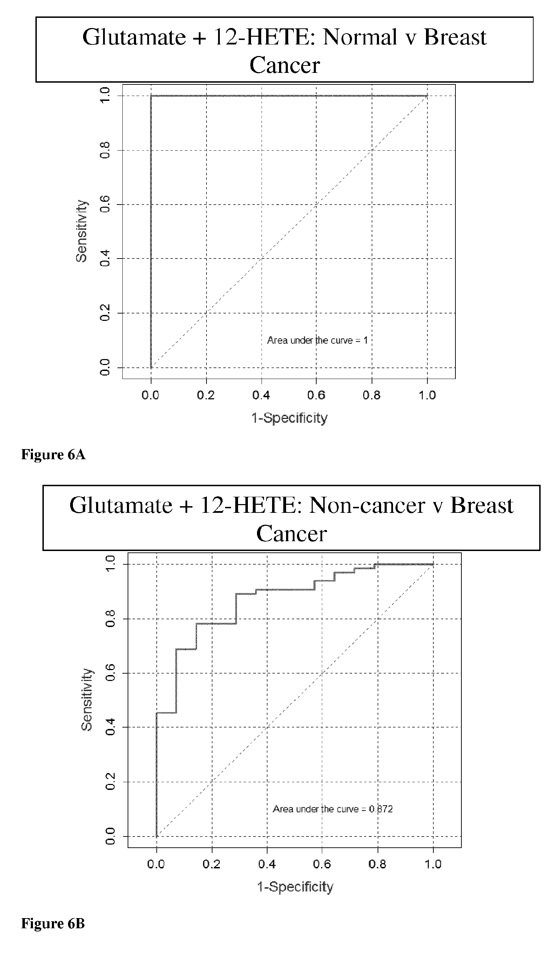

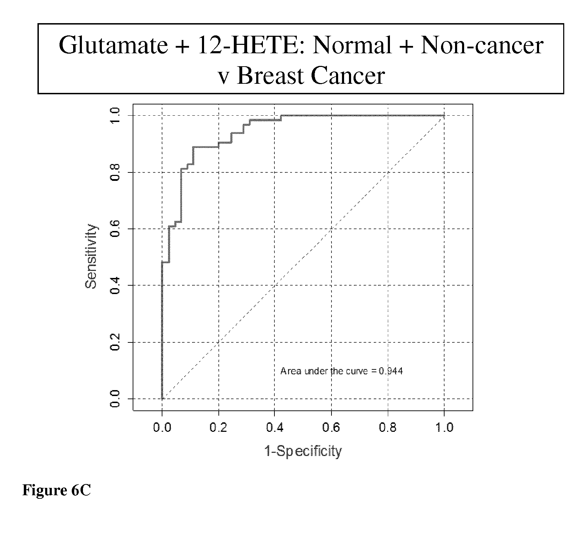

**Figure 6A-6C** illustrate AUC curves for (A) normal, (B) non-cancer, and (C) normal and non-cancer v. breast cancer for Glutamate and 12 HETE combined.

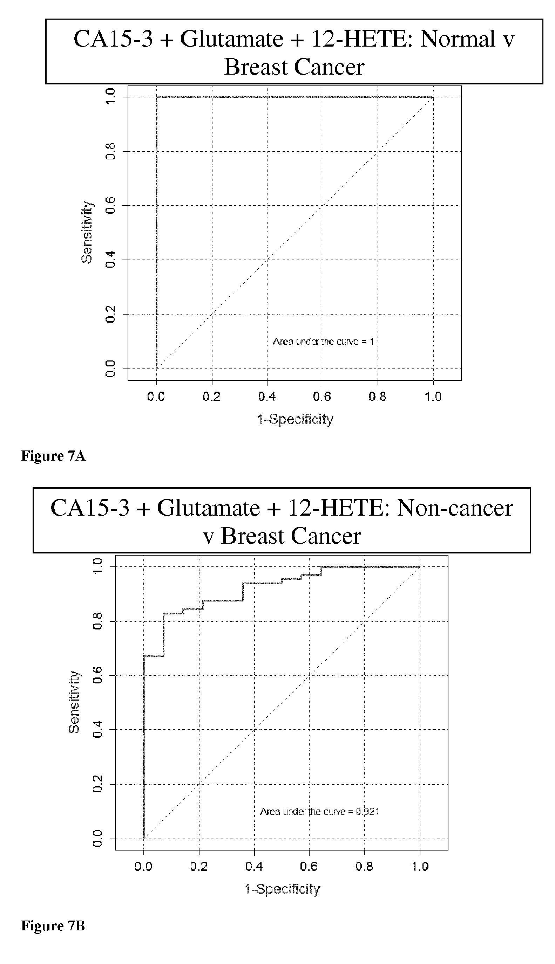

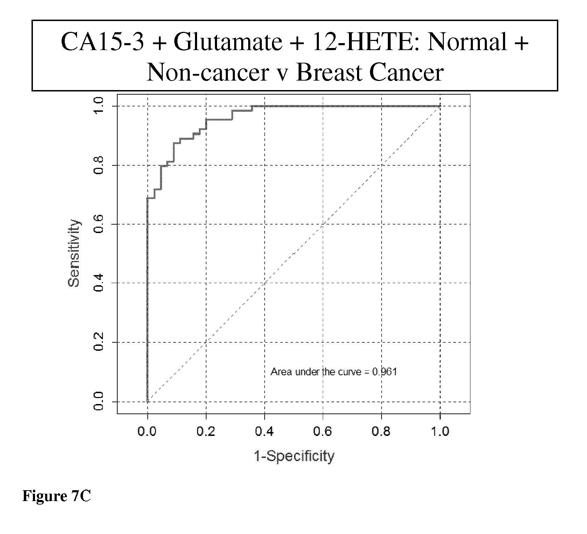

**Figure 7A-7C** illustrate AUC curves for (A) normal, (B) non-cancer, and (C) normal and non-cancer v. breast cancer for CA 15-3, Glutamate and 12 HETE combined.

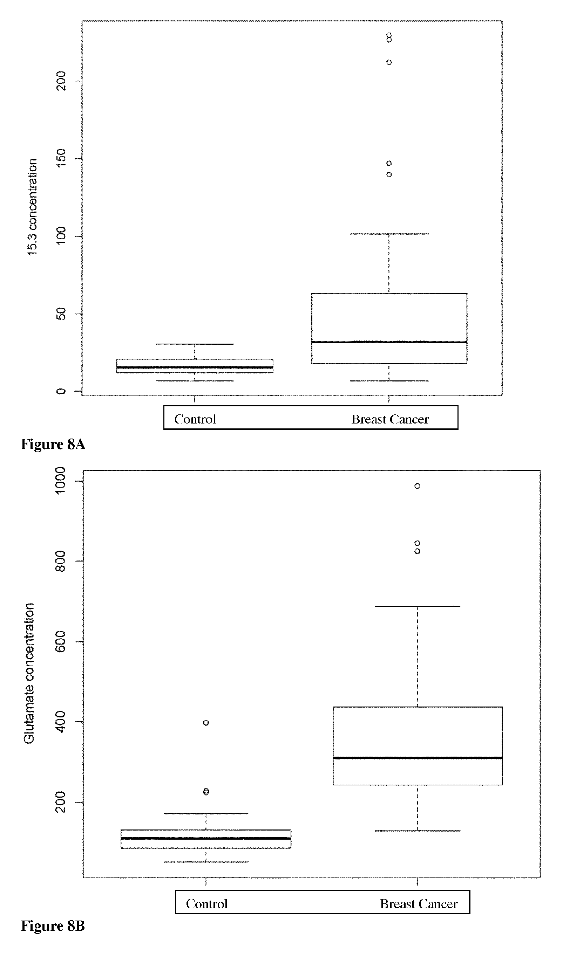

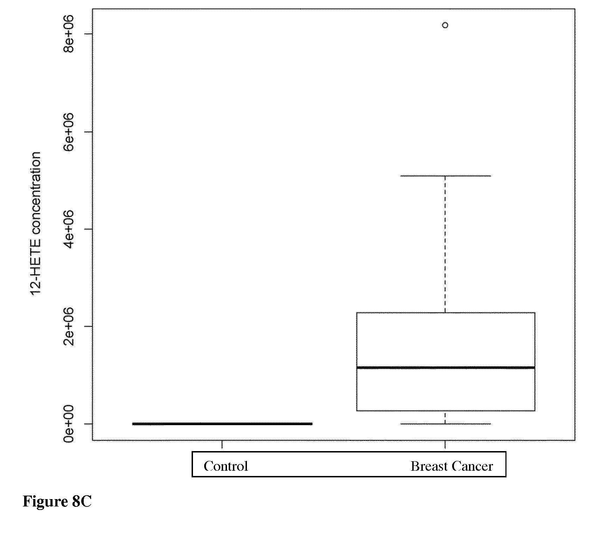

**Figure 8A-8C** illustrate box and whisker plots demonstrating the significant difference between normal v. breast cancer for all 3 proteins, CA 15-3, Glutamate and 12 HETE.

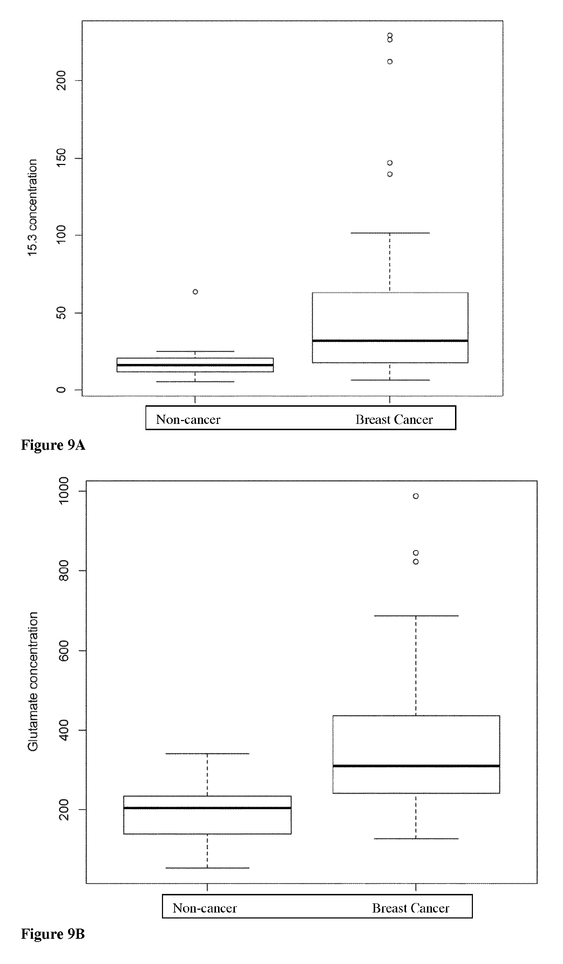

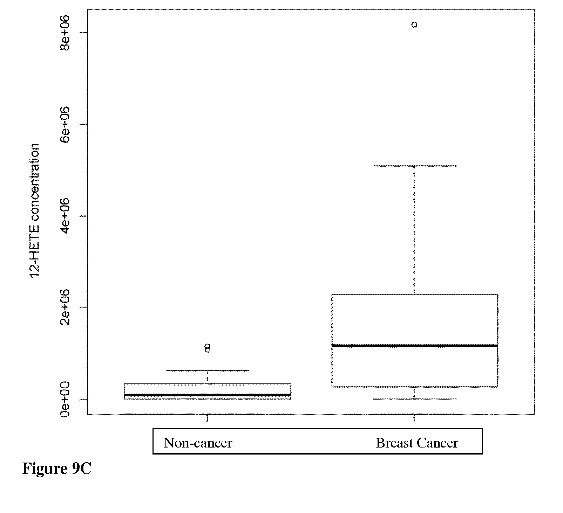

**Figure 9A-9C** illustrate box and whisker plots demonstrating the significant difference between non-cancer v. breast cancer for all 3 proteins, CA 15-3, Glutamate and 12 HETE.

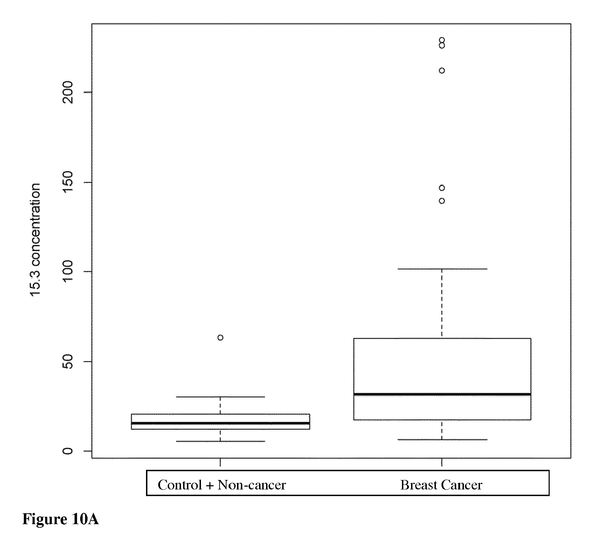

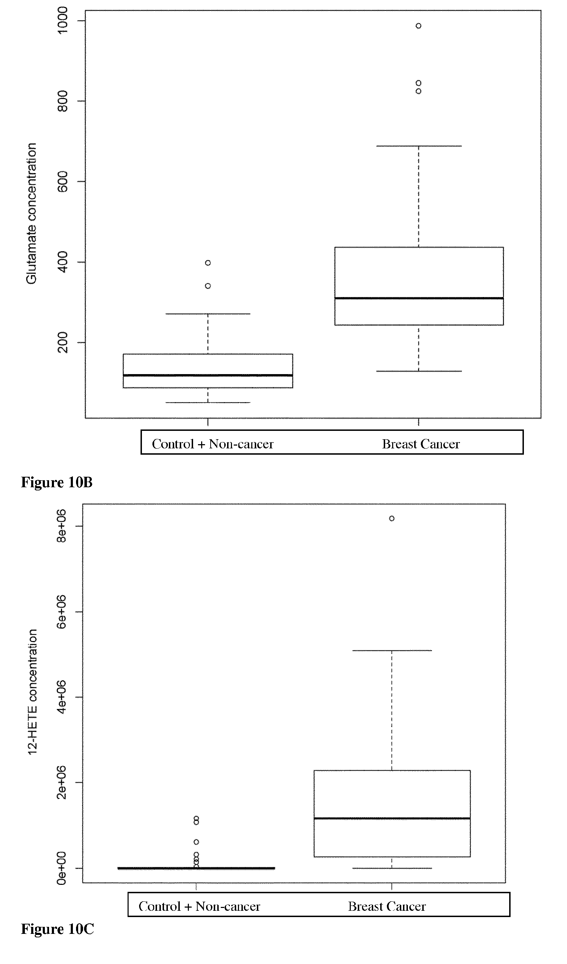

**Figure 10A-10C** illustrate box and whisker plots demonstrating the significant difference between normal and non-cancer v. breast cancer for all 3 proteins, CA 15-3, Glutamate and 12 HETE.

**Figure 11A-11C** illustrate bar charts demonstrating the significant difference between normal v. the three stages of breast cancers for all 3 proteins, CA 15-3, Glutamate and 12 HETE.

## Detailed Description of the Drawings

## Materials and Methods

Serum Sample Collection:

1. Put approx. 10 ml blood into serum tube.
2. Allow the blood to clot for 30 minutes to 1 hour at room temperature.
3. Clarify by centrifugation at 1000 x g for 15 minutes at room temperature.
4. Remove clarified serum from the pellet and dispense (as approx. 1 ml volumes X 3) into labelled cryovial tubes
5. If possible snap freeze samples immediately
6. Store samples frozen at -80°C until required for use.

## CA 15-3 Enzyme-linked Immunosorbent Assay (ELISA)

CA 15-3 is an epitope of a large transmembrane glycoprotein named MUC1 that is derived from the *MUC1* gene. The MUC1 protein, also known as polymorphic epithelial mucin or epithelial membrane antigen, has a large extracellular region, a transmembrane sequence, and a cytosolic domain. This protein is frequently overexpressed and aberrantly glycosylated on its extracellular region in breast cancer. CA15-3 is most useful for monitoring patients post-operatively for recurrence, particularly metastatic diseases. 96% of patients with local and systemic recurrence have elevated CA15-3, which can be used to predict recurrence earlier than radiological and clinical criteria. A 25% increase in the serum CA15-3 is associated with progression of carcinoma. A 50% decrease in serum CA15-3 is associated with response to treatment. CA15-3 is more sensitive than Carcinoembryonic Antigen (CEA) in early detection of breast cancer recurrence.

1. The CA 15-3 ELISA is based on the principle of a solid phase ELISA. The assay system utilizes two unique antibodies directed against distinct antigenic determinants on the intact CA 15-3 molecule.
2. A monoclonal antibody directed against a distinct antigenic determinant on the intact CA 15-3 molecule is used for solid phase immobilization (on the microtiter wells). A rabbit anti-CA 15-3 antibody conjugated to horseradish peroxidase (HRP) is in the antibody-enzyme conjugate solution.
3. The test sample is allowed to react sequentially with the two antibodies, resulting in the CA 15-3 molecules being sandwiched between the solid phase and enzyme-linked antibodies. After two separate 1-hour incubation steps at 37°C, the wells are washed with Wash Buffer to remove unbound labelled antibodies.
4. A solution of tetramethylbenzidine (TMB) reagent is added and incubated for 20 minutes, resulting in the development of a blue colour.
5. The colour development is stopped with the addition of 1N hydrochloric acid (HCl) changing the colour to yellow. The concentration of CA 15-3 is directly proportional to the colour intensity of the test sample. Absorbance is measured spectrophotometrically at 450 nm.

## Glutamate Assay

Glutamate (Glutamic acid: abbreviated as Glu or E) is one of the 20-22 proteinogenic amino acids, and its codons are GAA and GAG. It is a non-essential amino acid. Glutamate is a key compound in cellular metabolism. In humans, dietary proteins are broken down by digestion into amino acids, which serve as metabolic fuel for other functional roles in the body. Glutamate is the most abundant swift excitatory neurotransmitter in the mammalian nervous system. It is believed to be involved in learning and memory and has appeared to be involved in diseases like amyotrophic lateral sclerosis, lathyrism, autism, some forms of mental retardation and Alzheimer's disease. Glutamic acid is also present in a wide variety of foods, and has been used as a flavour enhancer in food industry. This Glutamate Assay Kit provides a sensitive detection method of the glutamate in a variety of samples.

The Glutamate Enzyme Mix recognizes glutamate as a specific substrate leading to proportional colour development. The amount of glutamate can therefore be easily quantified by colorimetric (spectrophotometry at $\lambda = 450$ nm) method.

## 12-HETE ELISA

12(S)-HETE is the stereospecific hydroxy product from the reduction of 12(S)-hydroperoxy tetraenoic eicosatetraenoic acid [12(S)-HpETE], which itself is a 12-lipoxygenase metabolite of arachidonic acid. 12(S)-HETE has been shown to be chemotactic and chemokinetic for polymorphonuclear leukocytes and vascular smooth cells. It also acts as a second messenger in angiotensin-II induced aldosterone production. Evidence also suggests that 12(S)-HETE is involved in suppressing renin production, stimulating insulin secretion by pancreatic tissue, inducing endothelial cell retraction and tumour cell adhesion.

1. Standards and samples are added to wells coated with a GxR IgG antibody. A blue solution of 12(S)-HETE conjugated to alkaline phosphatase is then added, followed by a yellow solution of rabbit polyclonal antibody to 12(S)-HETE.
2. During a simultaneous incubation at room temperature the antibody binds, in a competitive manner, the 12(S)-HETE in the sample or conjugate. The plate is washed, leaving only bound 12(S)-HETE.
3. para-Nitrophenylphosphate (pNpp) substrate solution is added. The substrate generates a yellow colour when catalyzed by the alkaline phosphatase on the 12(S)-HETE conjugate.
4. Stop solution is added. The yellow colour is read at 405 nm. The amount of signal is indirectly proportional to the amount of 12(S)-HETE in the sample.

The figures described below show AUC values for CA 15-3, Glutamate and 12 HETE on their own. AUC values are also shown for a combination of CA 15-3 with Glutamate and CA 15-3 with 12-HETE. Finally, the combination of CA 15-3, Glutamate and 12-HETE is also included.

The area under the Receiver Operating Characteristic (ROC) curve (AUC) is widely recognized as the measure of a diagnostic test's discriminatory power. The maximum value for the AUC is 1.0, thereby indicating a (theoretically) perfect test (*i.e.*, 100% sensitive and 100% specific). An AUC value of 0.5 indicates no discriminative value (*i.e.*, 50% sensitive and 50% specific) and is represented by a straight, diagonal line extending from the lower left corner to the upper right. The general rules or guidelines for interpreting AUC values are as follows:

0.5 = No discrimination (*i.e.*, indeterminate)
0.7 - 0.79 = Acceptable or fair discrimination
0.8 - 0.89 = Fair discrimination
$\geq$ 0.9 = Outstanding discrimination

Assays were performed on serum samples from Controls (disease-free individuals and individuals with non-cancer breast disease) compared to a group of patients with metastatic breast cancer. In total, 45 control samples and 85 metastatic breast cancer samples were analysed. Assay for CA 15-3 and 12-HETE were by ELISA. Assay for glutamate was an enzyme-based colorimetric assay, but ELISA is also available.

*Definitions*

In the specification, the term "Treatment" or "therapy" is generally accepted to encompasses prohibiting, preventing, restraining, and slowing, stopping or reversing progression or severity of a breast cancer. Examples of therapies include surgery, radiation, treatment with chemical agents (*e.g.* taxanes, anthracyclines, platinum-based drugs), and newer targeted agents such as erastuzumab and lapatinib, and other treatments known to those skilled in the art.

In this specification, the term "non-responsive", in relation to treating a breast cancer, should be understood to mean that a breast cancer is not responding to either a first-line therapy or second-line therapy, or both. First-line therapy is considered the first treatment given for a disease. It is often part of a standard set of treatments, such as surgery followed by chemotherapy and radiation. When used by itself, first-line therapy is the one accepted as the best treatment. If the first-line therapy does not appear to be having an effect, or does not agree with the patient, second-line therapy is considered which differs from the treatment given initially.

"Breast cancer patient" or "patient": This term means a patient who has a primary breast cancer tumour and awaits treatment for the cancer or has already undergone or is undergoing treatment for the primary tumour. The term should also be understood to include a patient who has had a primary breast cancer and is in remission, for example remission following treatment including one or more of tumour resection, first line chemotherapy, or both. Usually, the patient will be a breast cancer patient who has, or is undergoing, treatment for a primary tumour and who has been identified as having potential for developing a metastatic phenotype. In one embodiment, the patient has an ER-positive breast cancer.

In this specification, the term "biological sample" or "sample" may be any sample obtained from an individual such as, for example, blood, serum, saliva, urine, cerebrospinal fluid, tissue, cells, breast cancer tumour specimen, *etc.* Suitably, the biological sample or sample will be serum or breast cancer tumour specimen. Ideally, the biological sample will be serum.

In this specification, the term "monitoring breast cancer patients' response to treatment" includes monitoring response to a treatment and determining the effectiveness of treatment in the patient.

In the specification, the term "reference expression levels for the biomarkers" should be understood to mean reference expression levels of the biomarkers in one or more patients that do not have breast cancer or breast disease. Thus, when the method of the invention involves detecting changed expression of CA 15-3 and glutamate, the reference expression level for CA 15-3 will be the expression levels of CA15-3 in one or more patients that do not have breast cancer or breast disease, and the reference expression level for glutamate will be the expression levels of glutamate in one or more patients that do not have breast cancer or breast disease

Embodiments of the invention also provide for systems (and computer readable media for causing computer systems) to perform a method for diagnosing metastatic potential of a breast cancer in an individual.

Embodiments of the invention can be described through functional modules, which are defined by computer executable instructions recorded on computer readable media and which cause a computer to perform method steps when executed. The modules are segregated by function for the sake of clarity. However, it should be understood that the modules/systems need not correspond to discreet blocks of code and the described functions can be carried out by the execution of various code portions stored on various media and executed at various times. Furthermore, it should be appreciated that the modules may perform other functions, thus the modules are not limited to having any particular functions or set of functions.

The computer readable storage media can be any available tangible media that can be accessed by a computer. Computer readable storage media includes volatile and non-volatile, removable and non-removable tangible media implemented in any method or technology for storage of information such as computer readable instructions, data structures, program modules or other data. Computer readable storage media includes, but is not limited to, RAM (random access memory), ROM (read only memory), EPROM (erasable programmable read only memory), EEPROM (electrically erasable programmable read only memory), flash memory or other memory technology, CD-ROM (compact disc read only memory), DVDs (digital versatile disks) or other optical storage media, magnetic cassettes, magnetic tape, magnetic disk storage or other magnetic storage media, other types of volatile and non-volatile memory, and any other tangible medium which can be used to store the desired information and which can accessed by a computer including and any suitable combination of the foregoing.

Computer-readable data embodied on one or more computer-readable storage media may define instructions, for example, as part of one or more programs that, as a result of being executed by a computer, instruct the computer to perform one or more of the functions described herein, and/or various embodiments, variations and combinations thereof. Such instructions may be written in any of a plurality of programming languages, for example, Java, J#, Visual Basic, C, C#, C++, Fortran, Pascal, Eiffel, Basic, COBOL assembly language, and the like, or any of a variety of combinations thereof. The computer-readable storage media on which such instructions are embodied may reside on one or more of the components of either of a system, or a computer readable storage medium described herein, may be distributed across one or more of such components.

The computer-readable storage media may be transportable such that the instructions stored thereon can be loaded onto any computer resource to implement the aspects of the present invention discussed herein. In addition, it should be appreciated that the instructions stored on the computer-readable medium, described above, are not limited to instructions embodied as part of an application program running on a host computer. Rather, the instructions may be embodied as any type of computer code (e.g., software or microcode) that can be employed to program a computer to implement aspects of the present invention. The computer executable instructions may be written in a suitable computer language or combination of several languages. Basic computational biology methods are known to those of ordinary skill in the art and are described in, for example, Setubal and Meidanis et al., Introduction to Computational Biology Methods (PWS Publishing Company, Boston, 1997); Salzberg, Searles, Kasif, (Ed.), Computational Methods in Molecular Biology, (Elsevier, Amsterdam, 1998); Rashidi and Buehler, Bioinformatics Basics: Application in Biological Science and Medicine (CRC Press, London, 2000) and Ouelette and Bzevanis Bioinformatics: A Practical Guide for Analysis of Gene and Proteins (Wiley & Sons, Inc., 2nd ed., 2001).

The functional modules of certain embodiments of the invention include at minimum a determination system, a storage device, optionally a comparison module, and a display module. The functional modules can be executed on one, or multiple, computers, or by using one, or multiple, computer networks. The determination system has computer executable instructions to provide *e.g.*, CA 15-3, Glutamate and/or 12 HETE expression levels in computer readable form.

The determination system, can comprise any system for assaying a breast cancer tumor sample, or serum sample from a breast cancer patient, for expression of CA 15-3, Glutamate and/or 12 HETE. Standard procedures, such as immunohistochemistry or an enzyme-linked immunosorbent assay (ELISA), or an enzyme-based colorimetric assay, may be employed.

The information determined in the determination system can be read by the storage device. As used herein the "storage device" is intended to include any suitable computing or processing apparatus or other device configured or adapted for storing data or information. Examples of an electronic apparatus suitable for use with the present invention include a stand-alone computing apparatus, data telecommunications networks, including local area networks (LAN), wide area networks (WAN), Internet, Intranet, and Extranet, and local and distributed computer processing systems. Storage devices also include, but are not limited to: magnetic storage media, such as floppy discs, hard disc storage media, magnetic tape, optical storage media such as CD-ROM, DVD, electronic storage media such as RAM, ROM, EPROM, EEPROM and the like, general hard disks and hybrids of these categories such as magnetic/optical storage media. The storage device is adapted or configured for having recorded thereon nucleic acid sequence information. Such information may be provided in digital form that can be transmitted and read electronically, e.g., via the Internet, on diskette, via USB (universal serial bus) or via any other suitable mode of communication.

As used herein, "stored" refers to a process for encoding information on the storage device. Those skilled in the art can readily adopt any of the presently known methods for recording information on known media to generate manufactures comprising information relating to CA 15-3, Glutamate and/or 12 HETE expression in a sample.

In one embodiment the reference data stored in the storage device to be read by the comparison module is compared.

The "comparison module" can use a variety of available software programs and formats for the comparison operative to compare CA 15-3, Glutamate and/or 12 HETE expression information data determined in the determination system to reference samples and/or stored reference data. In one embodiment, the comparison module is configured to use pattern recognition techniques to compare information from one or more entries to one or more reference data patterns. The comparison module may be configured using existing commercially-available or freely-available software for comparing patterns, staining, and may be optimized for particular data comparisons that are conducted. The comparison module provides computer readable information related to the CA 15-3, Glutamate and/or 12 HETE expression levels of the sample.

The comparison module, or any other module of the invention, may include an operating system (*e.g.*, UNIX) on which runs a relational database management system, a World Wide Web application, and a World Wide Web server. World Wide Web application includes the executable code necessary for generation of database language statements (e.g., Structured Query Language (SQL) statements). Generally, the executables will include embedded SQL statements. In addition, the World Wide Web application may include a configuration file which contains pointers and addresses to the various software entities that comprise the server as well as the various external and internal databases which must be accessed to service user requests. The Configuration file also directs requests for server resources to the appropriate hardware--as may be necessary should the server be distributed over two or more separate computers. In one embodiment, the World Wide Web server supports a TCP/IP protocol. Local networks such as this are sometimes referred to as "Intranets." An advantage of such Intranets is that they allow easy communication with public domain databases residing on the World Wide Web (*e.g.*, the GenBank or Swiss Pro World Wide Web site). Thus, in a particular preferred embodiment of the present invention, users can directly access data (via Hypertext links for example) residing on Internet databases using a HTML interface provided by Web browsers and Web servers.

The comparison module provides a computer readable comparison result that can be processed in computer readable form by predefined criteria, or criteria defined by a user, to provide a content based in part on the comparison result that may be stored and output as requested by a user using a display module.

In one embodiment of the invention, the content based on the comparison result or the determination system is displayed on a computer monitor. In one embodiment of the invention, the content based on the comparison result or determination system is displayed through printable media. The display module can be any suitable device configured to receive from a computer and display computer readable information to a user. Non-limiting examples include, for example, general-purpose computers such as those based on Intel PENTIUM-type processor, Motorola PowerPC, Sun UltraSPARC, Hewlett-Packard PA-RISC processors, any of a variety of processors available from Advanced Micro Devices (AMD) of Sunnyvale, California, or any other type of processor, visual display devices such as flat panel displays, cathode ray tubes and the like, as well as computer printers of various types.

In one embodiment, a World Wide Web browser is used for providing a user interface for display of the content based on the comparison result. It should be understood that other modules of the invention can be adapted to have a web browser interface. Through the Web browser, a user may construct requests for retrieving data from the comparison module. Thus, the user will typically point and click to user interface elements such as buttons, pull down menus, scroll bars and the like conventionally employed in graphical user interfaces.

The methods described herein therefore provide for systems (and computer readable media for causing computer systems) to perform methods as described in the Statements of Invention above, for example methods for monitoring the response (or lack thereof) of a breast cancer to various therapies, and of diagnosing the recurrence of breast cancer in a patient by measuring the levels of a combination of at least 2 biomarkers.

Systems and computer readable media described herein are merely illustrative embodiments of the invention for performing methods of monitoring response in an individual, and are not intended to limit the scope of the invention. Variations of the systems and computer readable media described herein are possible and are intended to fall within the scope of the invention.

The modules of the machine, or those used in the computer readable medium, may assume numerous configurations. For example, function may be provided on a single machine or distributed over multiple machines.

## Results

It was found that CA 15-3 has an AUC value of 0.8 for distinguishing the control group from the breast cancer group. Individually, both Glutamate and 12-HETE were found to have higher AUC values, 0.92 and 0.95, respectively. A combination of both CA 15-3 and Glutamate were found to have an AUC value of 0.95 while a combination of CA 15-3 and 12-HETE had an AUC value of 0.94 for distinguishing control from metastatic breast cancer samples. Glutamate and 12-HETE together had an AUC value of 0.94. The final combination of all three biomarkers, CA 15-3, Glutamate and 12-HETE had an AUC value of 0.96, which is a surprising and synergistic effect given the AUC values of the individual biomarkers and the AUC vales when the biomarkers were coupled. Thus, the combination of all three biomarkers represents the best combination of molecules for distinguishing control/normal samples from breast cancer samples.

Table 1: Table showing the mean concentration for CA 15-3, Glutamate and 12-HETE in normal, non-cancer (disease of the breast) and metastatic breast cancer.

| | Mean Concentration | Mean Concentration | Mean Concentration |
|---|---|---|---|
| | CA 15-3 | Glutamate | 12-HETE |
| *Normal | 16.54375 | 6.019608 | 3,390 |
| Non-Cancer | 15.74288 | 9.706735 | 29,8256 |
| Cancer | 19.98738 | 18.11063 | 1,702,513 |
| *Normal means "samples from normal, non-cancerous individuals, that is, the control sample". p-values as follows: Normal V Cancer: CA15-3 (p = 0.05271); Glutamate (p = 1.972e-10); 12-HETE (p < 2.2e-16). Non-cancer v Cancer: CA15-3 (p = 0.07); Glutamate (p = 0.001693); 12-HETE (p = 8.824e-5). | | | |

Table 2: Table showing AUC values for CA15.3, Glutamate and 12-HETE alone or in different combinations for control v breast cancer, non-cancer v breast cancer and control+non-cancer v breast cancer.

| | Normal vs. Cancer | Non-cancer vs. Cancer | Normal & Non-cancer vs. Cancer |
|---|---|---|---|
| CA 15-3 | 0.8089 | 0.7823 | 0.8006 |
| Glutamate | 0.9611 | 0.8364 | 0.9223 |
| 12-HETE | 1.0 | 0.8286 | 0.9467 |
| CA 15-3 + Glut | 0.9687 | 0.9017 | 0.9479 |
| CA 15-3 + 12 HETE | 1.0 | 0.8794 | 0.9350 |
| Glutamate + 12 HETE | 1.0 | 0.8716 | 0.9444 |
| CA 15-3 + Glutamate + 12 HETE | 1.0 | 0.9207 | 0.9614 |

**Mathematical Analysis**

A mathematical formula has been developed using logistic regression. The final output of this analysis is a model that can be used to determine probability of disease using an equation of the form:

$$p = \frac{1}{1+e^{-((\alpha \times marker)+(\beta \times marker2)+(\gamma \times marker3)+intercept)}} \qquad (1)$$

where,
marker 1 = CA 15-3 concentration;
marker 2 = Glutamate concentration, where the glutamate concentration can be zero when the concentration of 12 HETE is greater than zero but not when the 12 HETE concentration is zero;
marker 3 = 12-HETE concentration, where the 12 HETE concentration can be zero when the concentration of glutamate is greater than zero but not when the glutamate concentration is zero;
$\alpha$ = regression coefficient 1;
$\beta$ = regression coefficient 2;
$\gamma$ = regression coefficient 3; and
p = probability of cancer.

For tumour markers to be useful in all breast cancer patients, high levels of accuracy will be needed. CA 15-3 on its own has a reasonable level of accuracy for detecting breast cancer, however, it has been demonstrated by the inventors that by combining this widely used cancer marker with Glutamate and/or 12-HETE, or all three, the levels of accuracy increase from 0.8 to 0.96. This represents a significant improvement (and a synergistic effect relating to the use of all three biomarkers) in the ability to monitor the progression of breast cancer and also to monitor the effectiveness of treatment.

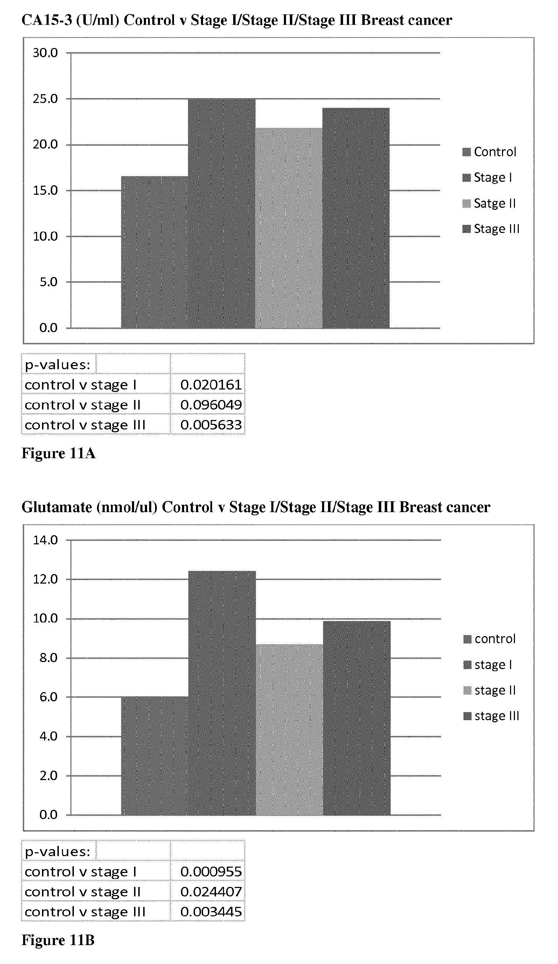

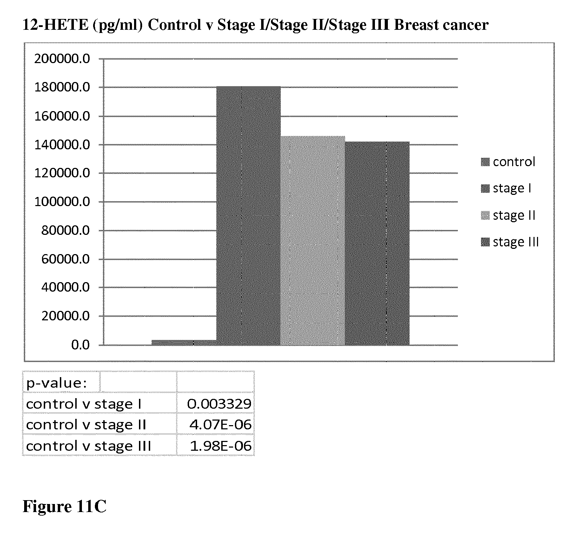

The results shown in Figures 11A, 11B and 11c illustrate that the 3 biomarkers are relevant to the detection of all stages of breast cancer.

The results presented herein clearly show that while glutamate on its own is indeed a good marker for monitoring a breast cancer patient's response to treatment, the AUC values, sensitivity and specificity of monitoring are considerably enhanced when the glutamate data is used in combination with one or both of the other markers CA 15-3 and 12-HETE.

In the specification the terms "comprise, comprises, comprised and comprising" or any variation thereof and the terms "include, includes, included and including" or any variation thereof are considered to be totally interchangeable and they should all be afforded the widest possible interpretation and vice versa.

The invention is not limited to the embodiments hereinbefore described but may be varied in both construction and detail.

**Claims**

1. A method of monitoring a breast cancer patient's response to treatment, the method comprising a step of:

   assaying a plurality of samples obtained from the patient undergoing breast cancer therapy over a period of time for expression levels of a plurality of biomarkers including Cancer Antigen 15-3 (CA 15-3) and one of either Glutamate or 12-Hydroxyeicosatetraenoic acid (12-HETE), and
   detecting a change in expression levels of CA 15-3 and one of either Glutamate or 12-HETE over the time period,

   wherein detection of a decreased expression levels of CA 15-3 and one of either Glutamate or 12-HETE over the time period is indicative of a response to the breast cancer therapy and wherein detection of increased or stable expression levels of CA 15-3 and one of either Glutamate or 12-HETE over the time period is indicative of a non-response to the breast cancer therapy.

2. A method for determining recurrence of cancer in a breast cancer patient, the method comprising a step of:

   assaying a plurality of samples obtained from the patient over a period of time for expression levels of a plurality of biomarkers including Cancer Antigen 15-3 (CA 15-3) and one of either Glutamate or 12-Hydroxyeicosatetraenoic acid (12-HETE), and
   detecting a change in expression levels of CA 15-3 and one of either Glutamate or 12-HETE when compared to reference expression levels for the biomarkers,

   wherein detection of increased expression levels of CA 15-3 and one of either Glutamate or 12-HETE when compared to the reference expression levels is indicative of a recurrence of cancer in the patient.

3. A method according to Claim 2, in which the biomarkers include CA 15-3 and both Glutamate and 12-HETE, wherein detection of increased expression levels of CA 15-3, Glutamate and 12-HETE, when compared with reference expression levels, is indicative of a risk of recurrence of breast cancer.

4. A method according to Claim 1, in which the biomarkers include CA 15-3 and both Glutamate and 12-HETE, wherein detection of stable or increased expression levels of CA 15-3, Glutamate and 12-HETE is indicative of a non-response to breast cancer therapy.

5. A method according to Claim 1 or Claim 4, in which the biomarkers include CA 15-3 and both Glutamate and 12-HETE, wherein detection of decreased expression levels of CA 15-3, Glutamate and 12-HETE is indicative of a response to breast cancer therapy.

6. A method according to any one of the preceding Claims having a discriminatory power (AUC value) of 0.94 or greater.

7. A method according to Claim 6 having a discriminatory power (AUC value) of 0.95 or greater.

8. A method according to Claim 6 in which the plurality of biomarkers include CA 15-3 and both Glutamate and 12-HETE, in which the method has a discriminatory power (AUC value) of 0.96 or greater.

9. A method according to any one of the preceding claims, in which the sample is selected from blood, serum, saliva, urine, cerebrospinal fluid, tissue, cells, breast cancer tumour specimen.

10. A method according to any one of the preceding claims, in which the expression levels are assayed by immunohistochemistry (IHC), enzyme-linked immunosorbent assay (ELISA), and other antibody-based methods which could be, for example, colorimetric, fluorimetric, potentiometric, or radiometric, or in the case of glutamate, an enzyme-based colorimetric assays.

11. A method as claimed in any one of the preceding Claims, in which the method is carried out periodically after surgery and/or during first line therapy.

12. A method as claimed in Claim 1, wherein detection of a decreased expression level of CA 15-3 and one of either Glutamate or 12-HETE relative to reference expression levels for the biomarkers over the time period is indicative of a response to breast cancer therapy and wherein detection of increased or stable expression levels of CA 15-3 and one of either Glutamate or 12-HETE relative to reference expression levels for the biomarkers over the time period is indicative of a non-response to the breast cancer therapy.

13. A method of treating or preventing breast cancer in a patient with a primary breast cancer, the method comprising the step of administering a second line therapy to the patient, wherein the patient is identified as having a non-responsive or a recurring breast cancer, using a method of any one of Claims 1 to 11.

14. A system for monitoring response to therapy and recurrence of breast cancer in an individual, the system comprising:

   a determination module configured to receive at least one test sample and perform at least one test analysis on the test sample to assay for expression of a plurality of biomarkers including CA 15-3 and one of either Glutamate or 12-HETE, or all three;
   optionally, a storage system configured to receive and store the expression data generated by the determination system for CA 15-3 and one of either Glutamate or 12-HETE, or all three;
   a comparison module for comparing the expression levels of the plurality of biomarkers with reference levels of the biomarkers and determining down-regulated, stable or increased expression of the biomarkers; and
   a display module for displaying a content based in part on the data output from said comparison module, wherein when stable or increased expression of CA 15-3 and one of either Glutamate or 12-HETE, or all three, is determined, the content comprises a signal indicative of a lack of response to therapy and/or recurrence of breast cancer.

15. A system as claimed in Claim 13 in which the determination system comprises a colorimetric detection apparatus or any apparatus which can quantitate specific antibody binding.

**Figure 1A**

**Figure 1B**

Figure 1C

## CA15-3: Non-cancer v Breast Cancer

**Figure 2A**

## Glutamate: Non-cancer v Breast Cancer

**Figure 2B**

12-HETE: Non-cancer v Breast Cancer

Area under the curve = 0.829

**Figure 2C**

CA15-3: Normal + Non-cancer v Breast Cancer

Area under the curve = 0.801

**Figure 3A**

Glutamate: Normal + Non-cancer v Breast Cancer

Area under the curve = 0.922

**Figure 3B**

12-HETE: Normal + Non-cancer v Breast
Cancer

Area under the curve = 0.947

Sensitivity

1-Specificity

**Figure 3C**

**CA15-3 + Glutamate: Normal v Breast Cancer**

Area under the curve = 0.969

Figure 4A

**CA15-3 + Glutamate: Non-cancer v Breast Cancer**

Area under the curve = 0.902

Figure 4B

**Figure 4C**

**Figure 5A**

**Figure 5B**

CA15-3 + 12-HETE: Normal + Non-cancer v Breast Cancer

Area under the curve = 0.935

Sensitivity

1-Specificity

**Figure 5C**

**Figure 6A**

**Figure 6B**

Glutamate + 12-HETE: Normal + Non-cancer v Breast Cancer

Figure 6C

## CA15-3 + Glutamate + 12-HETE: Normal v Breast Cancer

Figure 7A

## CA15-3 + Glutamate + 12-HETE: Non-cancer v Breast Cancer

Figure 7B

**Figure 7C**

Figure 8A

Figure 8B

**Figure 8C**

Figure 9A

Figure 9B

Figure 9C

**Figure 10A**

**Figure 10B**

**Figure 10C**

CA15-3 (U/ml) Control v Stage I/Stage II/Stage III Breast cancer

| p-values: | |
| --- | --- |
| control v stage I | 0.020161 |
| control v stage II | 0.096049 |
| control v stage III | 0.005633 |

Figure 11A

Glutamate (nmol/ul) Control v Stage I/Stage II/Stage III Breast cancer

| p-values: | |
| --- | --- |
| control v stage I | 0.000955 |
| control v stage II | 0.024407 |
| control v stage III | 0.003445 |

Figure 11B

**12-HETE (pg/ml) Control v Stage I/Stage II/Stage III Breast cancer**

| p-value: | |
|---|---|
| control v stage I | 0.003329 |
| control v stage II | 4.07E-06 |
| control v stage III | 1.98E-06 |

**Figure 11C**

## EUROPEAN SEARCH REPORT

Application Number

EP 14 15 4030

Europäisches
Patentamt
European
Patent Office
Office européen
des brevets

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | K.L. CHEUNG ET AL: "Tumour marker measurements in the diagnosis and monitoring of breast cancer", CANCER TREATMENT REVIEWS, vol. 26, no. 2, 1 April 2000 (2000-04-01), pages 91-102, XP055025947, ISSN: 0305-7372, DOI: 10.1053/ctrv.1999.0151 * abstract * | 1-12,14, 15 | INV. G01N33/574 |
| X | EP 0 727 664 A2 (BAYER AG [US]) 21 August 1996 (1996-08-21) * claims 1,3 * | 1-12,14, 15 | |
| X | CATHARINA WENZEL ET AL: "Preoperative second-line chemotherapy induces objective responses in primary breast cancer", WIENER KLINISCHE WOCHENSCHRIFT ; THE MIDDLE EUROPEAN JOURNAL OF MEDICINE, SPRINGER-VERLAG, VI, vol. 117, no. 1-2, 1 January 2005 (2005-01-01), pages 48-52, XP019376747, ISSN: 1613-7671, DOI: 10.1007/S00508-004-0282-Y * the whole document * | 13 | |
| X | JAMES COLIN R ET AL: "BRCA1, a potential predictive biomarker in the treatment of breast cancer", THE ONCOLOGIST, ALPHAMED PRESS, US, vol. 12, no. 2, 1 February 2007 (2007-02-01), pages 142-150, XP002516397, ISSN: 1083-7159, DOI: 10.1634/THEONCOLOGIST.12-2-142 * the whole document * | 13 | |

TECHNICAL FIELDS
SEARCHED (IPC)

G01N

-/--

The present search report has been drawn up for all claims

| Place of search | Date of completion of the search | Examiner |
|---|---|---|
| The Hague | 21 July 2014 | Wiesner, Martina |

Europäisches
Patentamt

European
Patent Office

Office européen
des brevets

**EUROPEAN SEARCH REPORT**

Application Number

EP 14 15 4030

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SUSANN BUSCH ET AL: "Low ERK Phosphorylation in Cancer-Associated Fibroblasts Is Associated with Tamoxifen Resistance in Pre-Menopausal Breast Cancer", PLOS ONE, vol. 7, no. 9, 24 September 2012 (2012-09-24), page e45669, XP055129353, ISSN: 1932-6203, DOI: 10.1371/journal.pone.0045669 * the whole document * | 1-13 | |
| A | MARCO TAMPELLINI ET AL: "Prognostic significance of changes in CA 15-3 serum levels during chemotherapy in metastatic breast cancer patients", BREAST CANCER RESEARCH AND TREATMENT, KLUWER ACADEMIC PUBLISHERS, BO, vol. 98, no. 3, 3 May 2006 (2006-05-03), pages 241-248, XP019392310, ISSN: 1573-7217, DOI: 10.1007/S10549-005-9155-Y * the whole document * | 1-15 | |
| A | TONDINI C ET AL: "COMPARISON OF CA15-3 AND CARCINOEMBRYONIC ANTIGEN IN MONITORING THE CLINICAL COURSE OF PATIENTS WITH METASTATIC BREAST CANCER", CANCER RESEARCH, AMERICAN ASSOCIATION FOR CANCER RESEARCH, US, vol. 48, no. 14, 15 July 1988 (1988-07-15), pages 4107-4112, XP000196353, ISSN: 0008-5472 * the whole document * | 1-15 | TECHNICAL FIELDS SEARCHED (IPC) |

-/--

The present search report has been drawn up for all claims

| Place of search | Date of completion of the search | Examiner |
|---|---|---|
| The Hague | 21 July 2014 | Wiesner, Martina |

EPO FORM 1503 03.82 (P04C01)

Europäisches
Patentamt

European
Patent Office

Office européen
des brevets

**EUROPEAN SEARCH REPORT**

Application Number

EP 14 15 4030

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SAFI F ET AL: "THE VALUE OF THE TUMOR MARKER CA 15-3 IN DIAGNOSING AND MONITORING BREAST CANCER. ÖA COMPARATIVE STUDY WITH CARCINOEMBRYONIC ANTIGEN", CANCER, AMERICAN CANCER SOCIETY, PHILADELPHIA, PA, US, vol. 68, no. 3, 1 August 1991 (1991-08-01), pages 574-582, XP000195878, ISSN: 0008-543X * the whole document * | 1-15 | |
| A | OLLENSCHLAEGER G ET AL: "PLASMA GLUTAMATE A PROGNOSTIC MARKER OF CANCER AND OF OTHER IMMUNODEFICIENCY SYNDROMES", SCANDINAVIAN JOURNAL OF CLINICAL AND LABORATORY INVESTIGATION, vol. 49, no. 8, 1989, pages 773-778, XP008170320, ISSN: 0036-5513 * the whole document * | 1-15 | |
| A | PANDEY A, RAMAKANT P, KUMAR S, SRIVASTAVA A, SRIVASTAVA M, ROY R: INTERNATIONAL SOCIETY FOR MAGNETIC RESONANCE IN MEDICINE, vol. 14, 2006, XP040596096, * the whole document * | 1-15 | TECHNICAL FIELDS SEARCHED (IPC) |
| A | YU LANFANG ET AL: "Analysis of urinary metabolites for breast cancer patients receiving chemotherapy by CE-MS coupled with on-line concentration", CLINICAL BIOCHEMISTRY, vol. 46, no. 12, 2013, pages 1065-1073, XP028679456, ISSN: 0009-9120, DOI: 10.1016/J.CLINBIOCHEM.2013.05.049 * the whole document * | 1-15 | |

The present search report has been drawn up for all claims

| Place of search | Date of completion of the search | Examiner |
|---|---|---|
| The Hague | 21 July 2014 | Wiesner, Martina |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

## ANNEX TO THE EUROPEAN SEARCH REPORT
## ON EUROPEAN PATENT APPLICATION NO.

EP 14 15 4030

This annex lists the patent family members relating to the patent documents cited in the above-mentioned European search report.
The members are as contained in the European Patent Office EDP file on
The European Patent Office is in no way liable for these particulars which are merely given for the purpose of information.

21-07-2014

| Patent document cited in search report | | Publication date | Patent family member(s) | | Publication date |
|---|---|---|---|---|---|
| EP 0727664 | A2 | 21-08-1996 | EP | 0727664 A2 | 21-08-1996 |
| | | | JP | H08248033 A | 27-09-1996 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SETUBAL ; MEIDANIS et al.** Introduction to Computational Biology Methods. PWS Publishing Company, 1997
- Computational Methods in Molecular Biology. Elsevier, 1998
- **RASHIDI ; BUEHLER.** Bioinformatics Basics: Application in Biological Science and Medicine. CRC Press, 2000
- **OUELETTE ; BZEVANIS.** Bioinformatics: A Practical Guide for Analysis of Gene and Proteins. Wiley & Sons, Inc, 2001